# EUROPEAN PATENT APPLICATION

(11) **EP 3 943 019 A1**
(43) Date of publication of application: **26.01.2022**
(21) Application number: 21186999.5
(22) Date of filing: 21.07.2021
(51) Int. Cl.: A61B 17/072, A61B 90/70

(54) **CARTRIDGE WITH CLEANING CAPABILITIES AND METHOD OF USE**

(30) Priority: 22.07.2020 US 202016935546
(71) Applicant: Covidien LP, Mansfield, MA 02048 (US)
(72) Inventor: BRONSON, Dwight, Cheshire, CT 06410 (US); CENICCOLA, Anthony L, Hamden, CT 06514 (US)
(74) Representative: Maschio, Antonio

(57) **Abstract**

A surgical stapling device includes a tool assembly having a cleaning cartridge, an anvil, and a drive member that supports a knife. The drive member is movable through a knife slot in the cleaning cartridge of the tool assembly to cut tissue clamped between the cleaning cartridge and the anvil. The cleaning cartridge includes a cleaner to clean the knife as the knife moves between its retracted and advanced positions to remove tissue cells from the knife.

## Description

### FIELD

The disclosure is directed to surgical devices for stapling tissue and, more particularly, to a cartridge or insert for use with a surgical stapling device with cleaning capabilities.

### BACKGROUND

Known surgical stapling devices include a tool assembly that has first and second jaw members that are movable in relation to each other between spaced and clamped positions. Typically, the first jaw member supports a staple cartridge and the second jaw member supports an anvil having staple-forming pockets. In order to reduce costs associated with a surgical procedure, the tool assembly and/or staple cartridge can be replaceable to facilitate reuse of the stapling device. In stapling devices in which only the cartridge assembly is replaced, the components of the tool assembly are reused during the surgical procedure at different locations within the body.

During a surgical procedure, stapling devices typically include a knife which is used to transect, resect, and anastomose healthy and diseased tissue. When transecting diseased tissue, there is the opportunity for diseased tissue cells to be captured or collected on the knife within the tool assembly. In stapling devices in which only the staple cartridge is replaced and the knife is reused, there is a concern that diseased tissue cells captured within the tool assembly will be translocated during reuse of the stapling device which may result in disease recurrence at a new location within the body.

A continuing need exists in the stapling arts for a stapling device that is adapted to minimize the likelihood of translocation of diseased tissue cells within the body of a patient during reuse of the surgical stapling device.

### SUMMARY

One aspect of this disclosure is directed to a cleaning cartridge that includes a body, a plurality of pushers, and a cleaning fluid. The body defines a central knife slot and a plurality of staple receiving slots positioned on each side of the central knife slot. Each pusher of the plurality of pushers is movable through one or more of the plurality of staple receiving slots. The cleaning fluid is supported in the body of the cleaning cartridge and is positioned to clean a knife moving through the central knife slot of the body.

Another aspect of the disclosure is directed to a tool assembly for a surgical stapling device that includes an anvil, a cartridge assembly, and a drive member. The cartridge assembly is coupled to the anvil such that the tool assembly is movable between an open position and a clamped position. The cartridge assembly includes a channel member and a cleaning cartridge. The channel member defines a channel and the cleaning cartridge is removably received within the channel of the channel member. The cleaning cartridge and the anvil define a tissue gap in the clamped position. The cleaning cartridge includes a body, a plurality of pushers, and a cleaning fluid. The body defines a central knife slot and a plurality of staple receiving slots on each side of the central knife slot. Each pusher of the plurality of pushers is movable through one or more of the plurality of staple receiving slots. The cleaning fluid is supported within the body of the cleaning cartridge. The drive member supports a knife and is movable between a retracted position and an advanced position to move the knife through the central knife slot. The cleaning fluid is positioned to clean the knife and the anvil as the knife moves through the central knife slot of the body.

In aspects of the disclosure, the cleaning fluid is positioned within the plurality of staple receiving slots and is forced from the plurality of staple receiving slots into the central knife slot by the plurality of pushers as the plurality of pushers move within the plurality of staple receiving slots.

In some aspects of the disclosure, protrusions are positioned on the body within the central knife slot and support the cleaning fluid.

In certain aspects of the disclosure, the cleaning fluid is coated on the protrusions.

In aspects of the disclosure, the protrusions are saturated with the cleaning fluid.

In some aspects of the disclosure, the protrusions are in the form of brushes, wipers, or sponges.

Another aspect of the disclosure is directed to a cleaning cartridge including a body, a plurality of pushers, and a cleaner. The body defines a central knife slot and a plurality of staple receiving slots on each side of the central knife slot. Each pusher of the plurality of pushers is movable through one or more of the plurality of staple receiving slots. The cleaner is supported within the body of the cleaning cartridge and is positioned to clean a knife moving through the central knife slot of the body.

Other features of the disclosure will be appreciated from the following description.

### BRIEF DESCRIPTION OF THE DRAWINGS

Various aspects of the disclosed surgical stapling device are described herein below with reference to the drawings, wherein:
FIG. 1 is a side perspective view of a surgical stapling device according to aspects of the disclosure with a tool assembly of the stapling device in an open position;
FIG. 2 is an enlarged view of the indicated area of detail shown in FIG. 1 with the tool assembly of the stapling device in the open position;
FIG. 3 is a side perspective view of the tool assembly shown in FIG. 2 with the tool assembly in the open position and a cleaning cartridge of a cartridge assembly removed from a channel of the cartridge assembly;
FIG. 4 is an exploded view of the cleaning cartridge of the tool assembly shown in FIG. 3;
FIG. 5 is a drive assembly of the tool assembly shown in FIG. 2;
FIG. 6 is an enlarged view of the indicated area of detail shown in FIG. 2;
FIG. 7 is a cross-sectional view taken along a longitudinal axis of the tool assembly shown in FIG. 2 with the tool assembly in a clamped position;
FIG. 8 is an enlarged view of the indicated area of detail shown in FIG. 7;
FIG. 9 is a side perspective view of the tool assembly shown in FIG. 7 with the tool assembly in the clamped position and the anvil shown in phantom;
FIG. 10 is a cross-sectional view taken along section line 10-10 of FIG. 9;
FIG. 11 is an enlarged view of the indicated area of detail shown in FIG. 10;
FIG. 12 is a cross-sectional view taken along section line 12-12 of FIG. 9;
FIG. 13 is a side cross-sectional view of another version of the tool assembly of the stapling device shown in FIG. 1; and
FIG. 14 is a cross-sectional view taken through the tool assembly of FIG. 13.

### DETAILED DESCRIPTION

The disclosed surgical stapling device will now be described in detail with reference to the drawings in which like reference numerals designate identical or corresponding elements in each of the several views. However, it is to be understood that the aspects of the disclosure described herein are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "endoscopic" is used generally to refer to endoscopic, laparoscopic, arthroscopic, and/or any other procedure conducted through small diameter incision or cannula. Further, the term "clinician" is used generally to refer to medical personnel including doctors, nurses, and support personnel.

A surgical stapling device is disclosed that includes a tool assembly having a cleaning cartridge, an anvil, and a drive member that includes a knife. The drive member is movable through a knife slot in the cleaning cartridge of the tool assembly to cut tissue clamped between the cleaning cartridge and the anvil. The cleaning cartridge includes a cleaner to clean the knife as the knife moves between its retracted and advanced positions to remove tissue cells from the knife to facilitate reuse of the surgical stapling device.

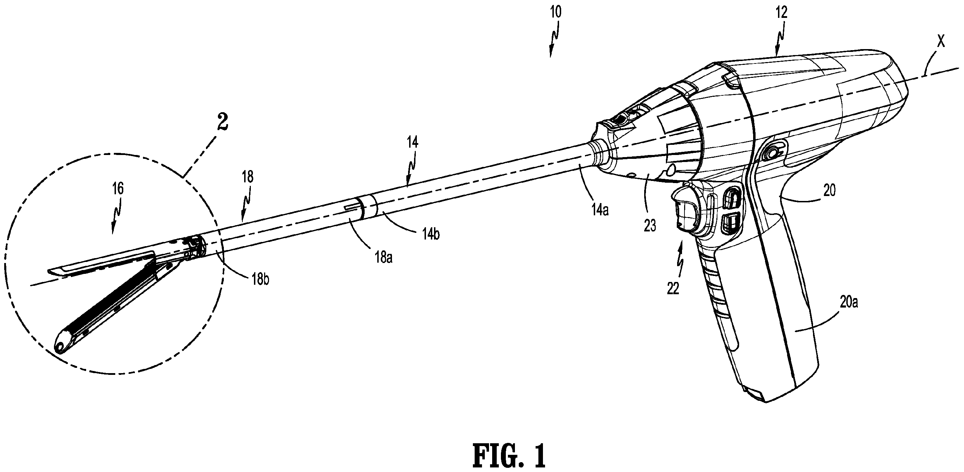

FIG. 1 is a perspective view of a surgical stapling device including aspects of the disclosure shown generally as stapling device 10. The stapling device 10 includes a handle assembly 12, an elongate body or adapter assembly 14, and a tool assembly 16. The adapter assembly 14 defines a longitudinal axis "X" and includes a proximal portion 14a that is supported on the handle assembly 12 and a distal portion 14b that supports the tool assembly 16. The proximal portion 14a of the adapter assembly 14 can be removably coupled to the handle assembly 12. In some aspects of the disclosure, the tool assembly 16 forms part of a reload assembly 18 that includes a proximal body portion 18a that is releasably coupled to the distal portion 14b of the adapter assembly 14 of the stapling device 10. The proximal body portion 18a of the reload assembly 18 includes a distal portion 18b that supports the tool assembly 16. In alternate aspects of the disclosure, the tool assembly 16 is fixedly secured to the distal portion 14b of the adapter assembly 14.

The handle assembly 12 of the stapling device 10 includes a body 20 that defines a stationary handle 20a, actuation buttons 22, and a rotation knob 23. The actuation buttons 22 can be depressed to actuate the tool assembly 16, e.g., approximate the tool assembly 16, articulate the tool assembly 16, fire staples, etc. The rotation knob 23 is rotatably supported on the body 20 of the handle assembly 12 and supports the adapter assembly 14 of the stapling device 10 such that rotation of the rotation knob 23 causes rotation of the adapter assembly 14 and the tool assembly 16 in relation to the handle assembly 12 about the longitudinal axis "X". In aspects of the disclosure, a battery (not shown) is supported in the stationary handle 20a to power the handle assembly 12. U.S. Patent No. 10,123,799 discloses a powered handle assembly and an adapter assembly suitable for use with the tool assembly 16.

It is envisioned that the stapling device 10 need not be powered but can also include a manually powered handle assembly such as described in U.S. Patent No. 5,865,361 ("the '361 patent"). It is also envisioned that the stapling device can be adapted to be supported on a robotic surgical system and need not include a handle assembly.

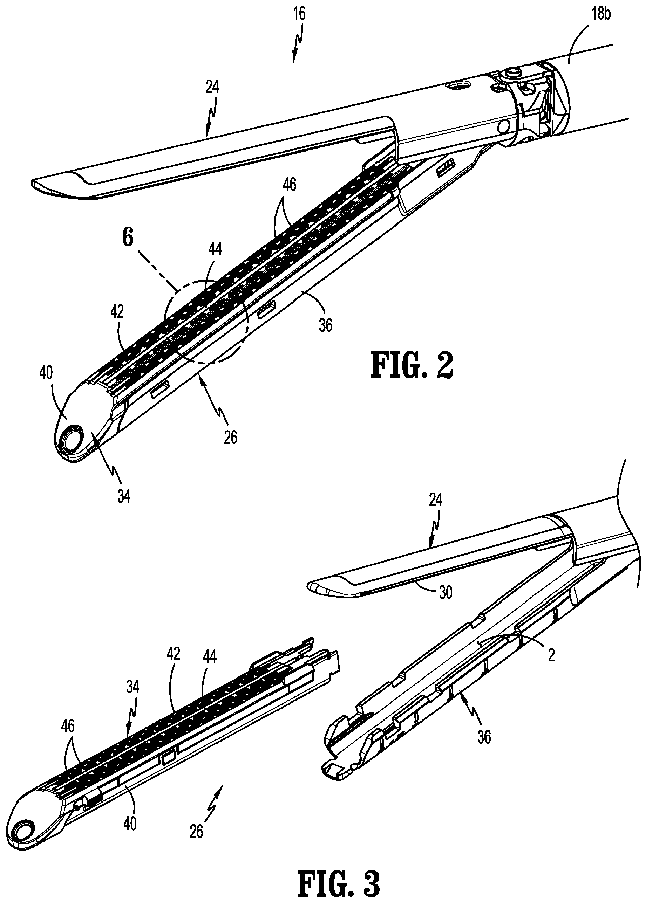

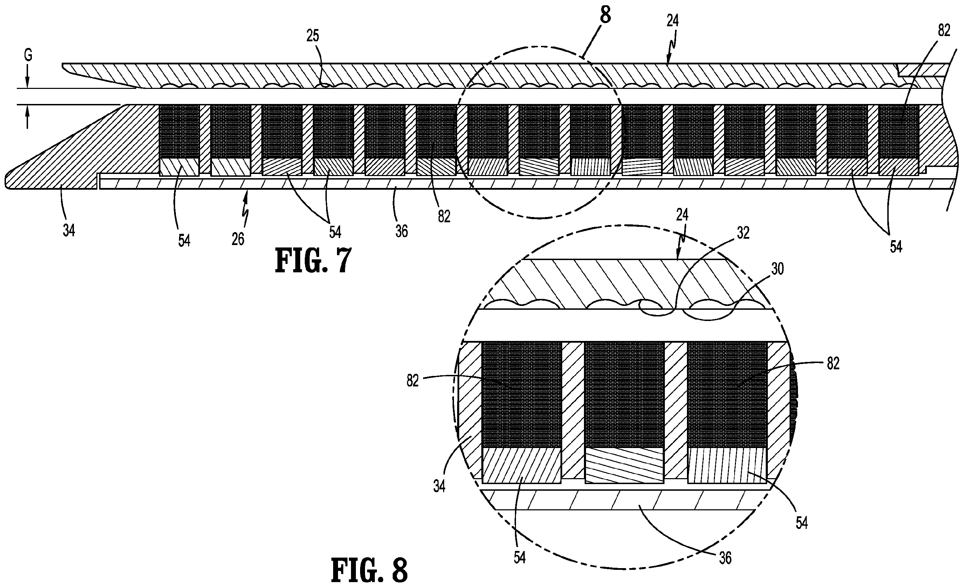

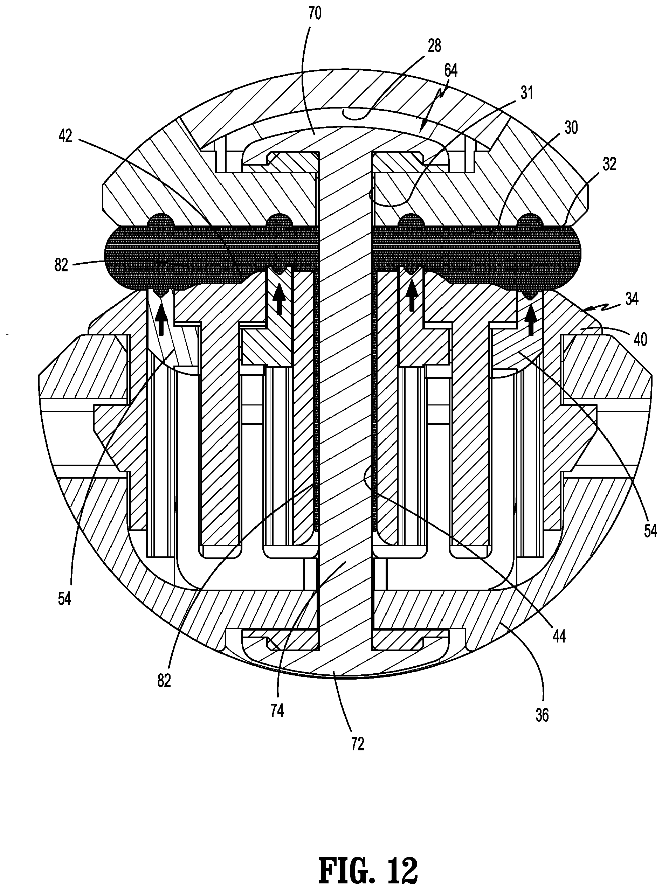

FIGS. 2-4 illustrate a distal portion of the stapling device 10 including the tool assembly 16 which includes an anvil 24 and a cartridge assembly 26. The anvil 24 and the cartridge assembly 26 are coupled together at their proximal ends such that the tool assembly 16 can move between an open position (FIG. 1) and a clamped position (FIG. 7). The anvil 24 defines a longitudinal channel 28 (FIG. 12) and includes a tissue engaging surface 30 (FIG. 8) that defines a plurality of staple forming pockets 32 (FIG. 8). The longitudinal channel 28 communicates with the tissue engaging surface 30 by a knife channel 31 (FIG. 12).

The cartridge assembly 26 includes a cleaning cartridge 34 and a channel member 36 that defines a channel 38 that receives the cleaning cartridge 34. In aspects of the disclosure, the cleaning cartridge 34 is removably received within the channel 38 defined by the channel member 36 to enable replacement of the cleaning cartridge 34 and facilitate reuse of the stapling device 10.

The cleaning cartridge 34 includes a body 40 that has a tissue engaging surface 42 and defines a central knife slot 44 and a plurality of staple receiving slots 46 that are positioned on each side of the central knife slot 44. In the open position of the tool assembly 16, a distal end portion of the anvil 24 is spaced from a distal end portion of the cartridge 34 to facilitate placement of tissue "T" (FIG. 7) between the anvil assembly 24 and the cartridge assembly 26. In the clamped position, the tissue engaging surface 30 of the anvil 24 is in juxtaposed alignment with the tissue engaging surface 42 of the cleaning cartridge 34 to define a tissue gap "G", and the staple forming pockets 32 of the anvil 24 are aligned with the staple receiving slots 46 of the cleaning cartridge 34. In aspects of the disclosure, the staple receiving slots 46 of the cleaning cartridge 34 are aligned in spaced rows positioned on each side of the central knife slot 42. Although three rows of staple receiving slots 46 are illustrated, it is envisioned that the body 40 of the cleaning cartridge 34 may have one or more rows of staple receiving slots 46 on each side of the central knife slot 42.

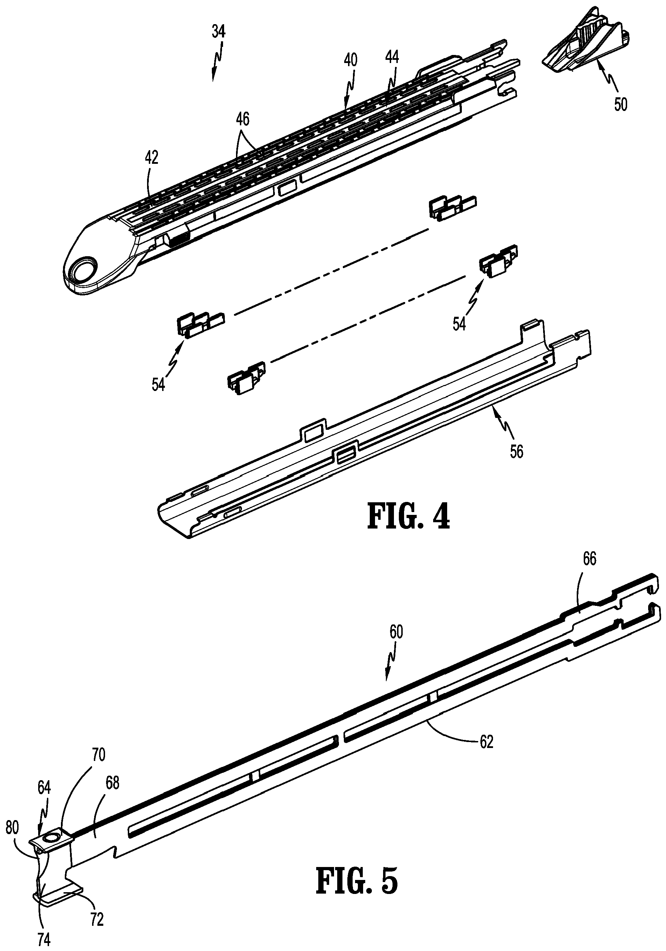

FIG. 4 illustrates an exploded view of the cleaning cartridge 34 which includes an actuation sled 50, pushers 54, and a pan 56. The pan 56 is secured to a lower portion of the body 40 and is positioned to retain the pushers 54 within the body 54. The actuation sled 50 is movable through the body 40 from a retracted position to an advanced position to drive the pushers 54 upwardly as viewed in FIG. 4. engaging surface 42 of the body 40. For a detailed description of an exemplary staple cartridge and its operation, see the '361 Patent.

FIG. 5 illustrates a drive member 60 of the tool assembly 16 of the stapling device 10. The drive member 60 includes a flexible beam 62 and a working end 64. The flexible beam 62 has a proximal end 66 that is adapted to engage a control rod (not shown) of the adapter assembly 14 (FIG. 1) and a distal end 68 that supports the working end 64. In aspects of the disclosure, the working end 64 of the drive member 60 includes an upper beam 70, a lower beam 72, and a vertical strut 74. The upper beam 70 is received within the channel 28 of the anvil 24. The lower beam 72 is positioned along an outer surface of the channel 36 of the cartridge assembly 26. The vertical strut 74 supports a knife 80 and extends through the knife channel 31 in the anvil 24 and the central knife slot 44 of the cleaning cartridge 34 across the tissue gap "G" (FIG. 7) defined between the anvil 24 and the cleaning cartridge 34. The knife 80 is positioned on the vertical strut 74 to extend across the tissue gap "G". The upper and lower beams 70 and 72 of the working end 64 of the drive member 60 engage the anvil 24 and the cartridge assembly 26 to limit the tissue gap "G" (FIG. 7) to a predetermined maximum distance when the tool assembly 16 is in the clamped position and the stapling device 10 (FIG. 1) is fired. The drive assembly 60 is moveable between a retracted position within the tool assembly to advance the actuation sled 50 (FIG. 4) through the body 40 of the cleaning cartridge 34 to move the pushers 54 within the body 40. For a detailed description of the operation of the drive member 60, see the '361 Patent.

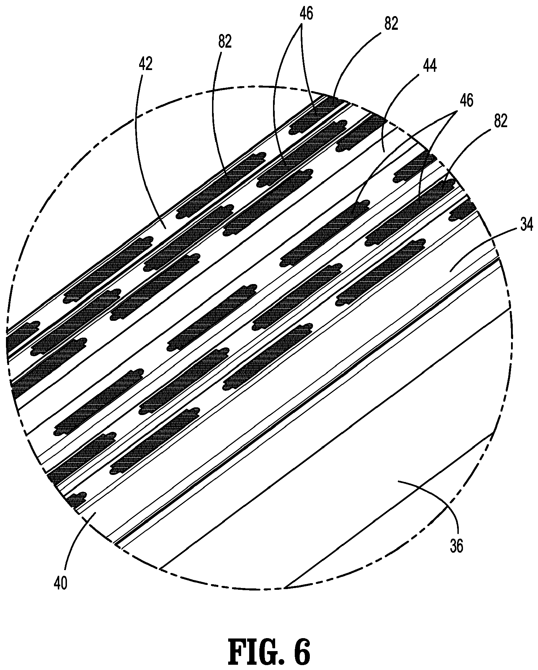

FIGS. 6-8 illustrate the staple receiving slots 46 of the body 40 of the cleaning cartridge 34. Each of the staple receiving slots 46 of the body 40 of the cleaning cartridge 34 is filled with a cleaning fluid 82. The lower ends of the staple receiving slots 46 is closed by the pushers 54. The cleaning fluid 82 surrounds is received on an upper surface of the pushers 54 as viewed in FIG. 8.

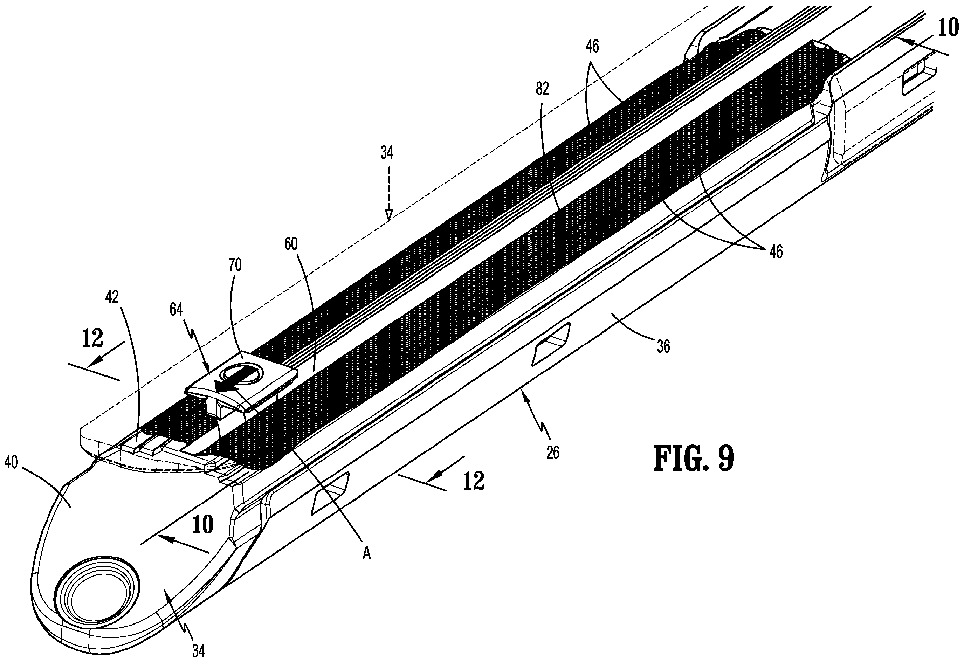

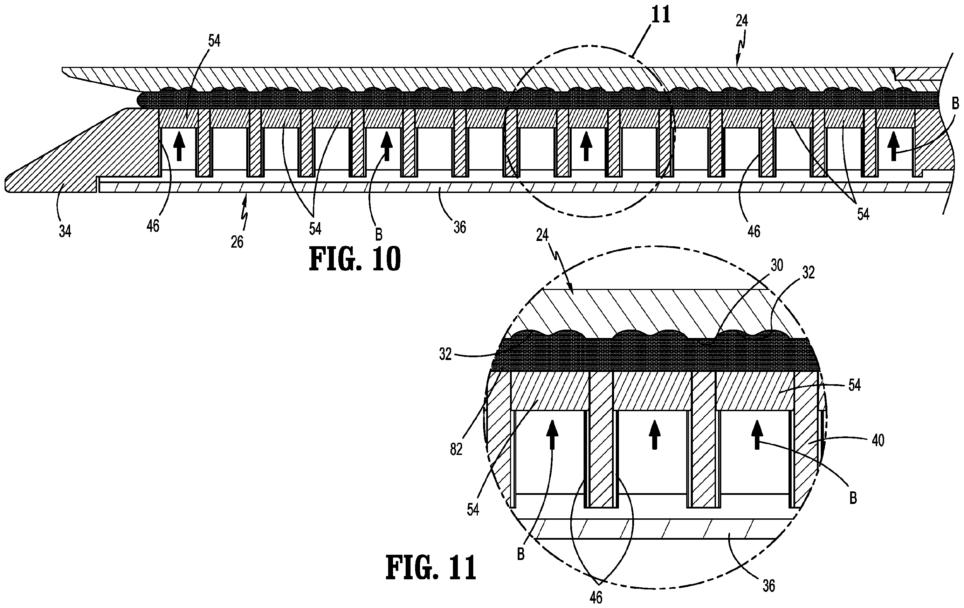

FIGS. 9-12 illustrate the tool assembly 16 as the stapling device 10 is fired. When the stapling device 10 (FIG. 1) is fired, the drive member 60 is moved through the tool assembly 16 from its retracted position (FIG. 7) to its advanced position (FIG. 10). As the drive member 60 is moved from its retracted position towards its advanced position in the direction of arrow "A" in FIG. 10, the working end 64 of the drive member 60 (FIG. 9) engages the actuation sled (FIG. 4) and advances the actuation sled 50 from its retracted position to its advanced position. As the actuation sled 50 moves through the body 40 and engages the pushers 54, the pushers 54 are moved upwardly towards the anvil 24 as viewed in FIGS. 10 and 11 in the direction of arrows "B". As the pushers 54 move upwardly, within the staple receiving slots 46, the cleaning fluid 82 is forced upwardly from the staple forming slots 46 and exits the staple forming slots 46 onto the tissue engaging surface 42 FIG. 9) of the body 40 of the cleaning cartridge 34. The cleaning fluid 82 is forced from the staple forming slots 46, onto and across the tissue engaging surface 42 of the body 40 of the cleaning cartridge 34, and into the central knife slot 44 (FIG. 12) of the cleaning cartridge 34. The cleaning fluid 82 flows to a position surrounding the vertical strut 74 and the knife 80 which is supported on the vertical strut 74 of the working end 64 of the drive member 60 as the tool assembly 16 is fired. The cleaning fluid 82 may also contact the anvil 24.

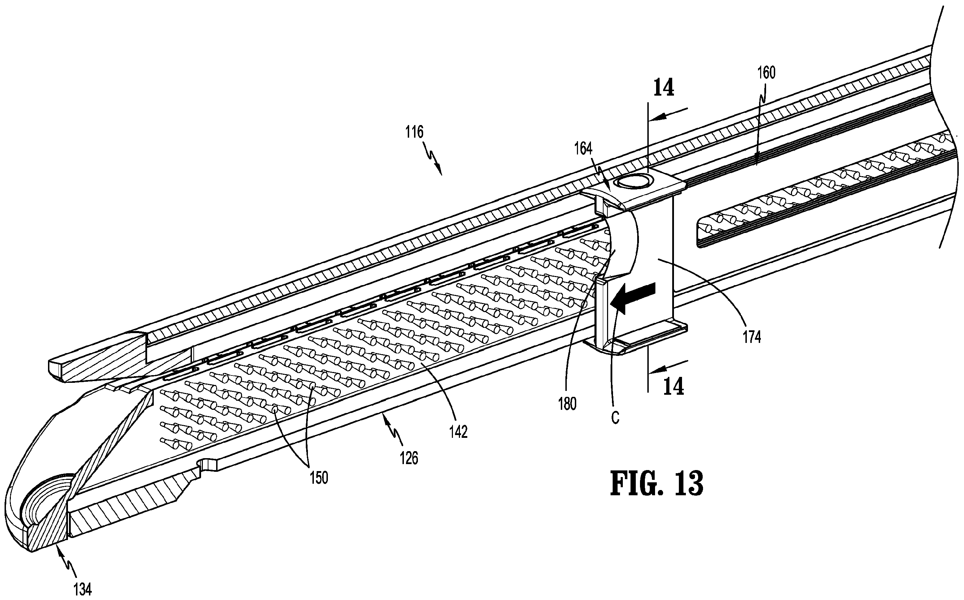

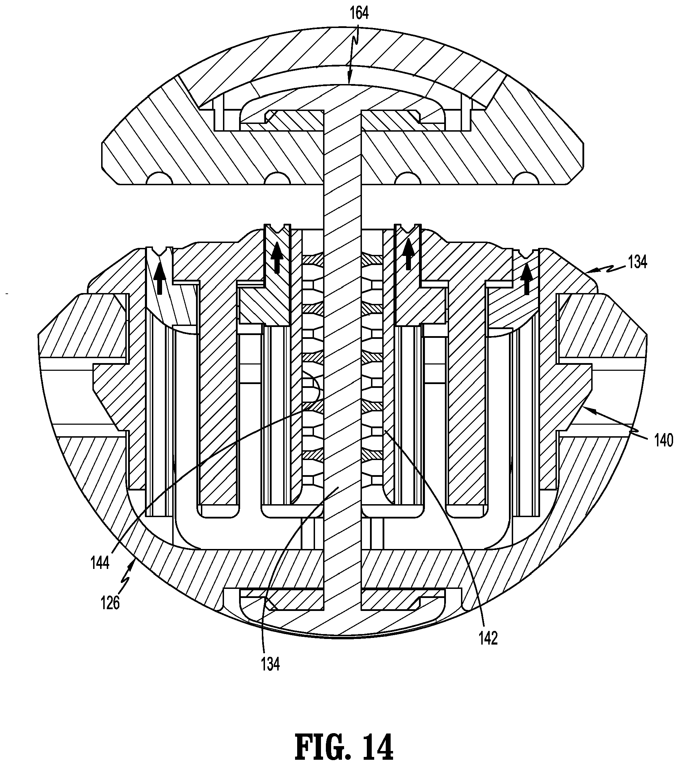

In the disclosed stapling device 10, the knife 80 is immersed in the cleaning fluid 82 to clean the knife components as the stapling device 10 is fired with the cleaning cartridge 34 supported within the channel member 36. The cleaning fluid 82 disinfects the knife 80 of the tool assembly 16 to minimize the likelihood of translocation of diseased tissue cells during reuse of the stapling device 10 (FIG. 1). In aspects of the disclosure, the cleaning fluid 82 can be selected from hydrogen peroxide, iodine/iodophors, alcohol solutions including ethyl alcohol and isopropyl alcohol, chlorhexidine gluconate, hypochlorites, quaternary ammonium compounds, etc. It is envisioned that the cleaning cartridge 34 can be inserted into the channel member 36 of the stapling device 10 between firings of the stapling device 10 to clean components of the stapling device 10.FIGS. 13 and 14 illustrate another version of the tool assembly of the disclosed stapling device shown generally as tool assembly 116. The tool assembly 116 is substantially like the tool assembly 16 except that the cleaning fluid is not received within the staple receiving slots 146 but rather is included on protrusions 150 supported on walls 142 of the body 140 defining the central knife slot 144 of the staple cartridge 134. More specifically, the walls 142 defining the central knife slot 144 (FIG. 14) of the staple cartridge 134 of the cartridge assembly 126 support protrusions 150 that extend across the central knife slot 144 into the path of the vertical strut 174 of the working end 164 of the drive member 160. In aspects of the disclosure, the protrusions 150 which may be in the form of brushes, sponges, wipers, or the like are coated or saturated with a cleaning fluid. It is envisioned that the protrusions may not include a cleaning fluid and serve only to scrape or wipe tissue cells from the knife 180 of the drive member 160.

When the stapling device 10 is fired as described above, the vertical strut 174 of the working end 164 of the drive member 160 moves through the central knife slot 144 (FIG. 14) of the staple cartridge 134 of the cartridge assembly 126 in the direction indicated by arrows "C". As the vertical strut 174 of the working end 164 of the drive member 160 moves through the central knife slot 144, the protrusions 150 engage the vertical strut 174 including the knife 180 to wipe tissue from the knife 180 and disinfect the knife 180 to facilitate reuse of the stapling device 10 (FIG. 1).

Persons skilled in the art will understand that the devices and methods specifically described herein and illustrated in the accompanying drawings are non-limiting exemplary aspects of the disclosure. It is envisioned that the elements and features illustrated or described in connection with one exemplary aspect of the disclosure may be combined with the elements and features of another without departing from the scope of the disclosure. As well, one skilled in the art will appreciate further features and advantages of the disclosure based on the above-described aspects of the disclosure. Accordingly, the disclosure is not to be limited by what has been particularly shown and described, except as indicated by the appended claims.

The invention may be described by reference to the following numbered paragraphs:-
1. A staple cartridge comprising:
   a body defining a central knife slot and a plurality of staple receiving slots on each side of the central knife slot;
      a plurality of pushers, each pusher of the plurality of pushers movable through one or more of the plurality of staple receiving slots; and
   a cleaning fluid supported in the body of the staple cartridge, the cleaning fluid positioned to clean a knife moving through the central knife slot of the body.
2. The staple cartridge of paragraph 1, wherein the cleaning fluid is positioned within the plurality of staple receiving slots and is forced from the plurality of staple receiving slots into the central knife slot by the plurality of pushers as the plurality of pushers move within the plurality of staple receiving slots.
3. The staple cartridge of paragraph 1, further including protrusions positioned on the body within the central knife slot, the protrusions supporting the cleaning fluid.
4. The staple cartridge of paragraph 3, wherein the cleaning fluid is coated on the protrusions.
5. The staple cartridge of paragraph 3, wherein the protrusions are saturated with the cleaning fluid.
6. The staple cartridge of paragraph 3, wherein the protrusions are in the form of brushes, wipers, or sponges.
7. A tool assembly for a surgical stapling device comprising:
   an anvil;
   a cartridge assembly coupled to the anvil such that the tool assembly is movable between an open position and a clamped position, the cartridge assembly including a channel member and a staple cartridge, the channel member defining a channel, the staple cartridge removably received within the channel of the channel member, the staple cartridge and the anvil defining a tissue gap in the clamped position, the staple cartridge including:
      a body defining a central knife slot and a plurality of staple receiving slots on each side of the central knife slot;
      a plurality of pushers, each pusher of the plurality of pushers movable through one or more of the plurality of staple receiving slots; and
      a cleaning fluid supported in the body of the staple cartridge; and
   a drive member supporting a knife, the drive member movable between a retracted position and an advanced position to move the knife through the central knife slot;
   wherein the cleaning fluid is positioned to clean the knife as the knife moves through the central knife slot of the body.
8. The tool assembly of paragraph 7, wherein the cleaning fluid is positioned within the plurality of staple receiving slots and is forced from the plurality of staple receiving slots into the central knife slot by the plurality of pushers as the plurality of pushers move within the plurality of staple receiving slots.
9. The tool assembly of paragraph 7, further including protrusions positioned on the body within the central knife slot, the protrusions supporting the cleaning fluid.
10. The tool assembly of paragraph 9, wherein the cleaning fluid is coated on the protrusions.
11. The tool assembly of paragraph 9, wherein the protrusions are saturated with the cleaning fluid.
12. The tool assembly of paragraph 9, wherein the protrusions are in the form of brushes, wipers, or sponges.
13. The tool assembly of paragraph 7, wherein the drive member includes a flexible beam and a working end, the working end including an upper beam, a lower beam, and a vertical strut, the vertical strut interconnecting the upper beam and the lower beam and supporting the knife.
14. The tool assembly of paragraph 13, wherein the anvil defines a channel that receives the upper beam.
15. The tool assembly of paragraph 14, wherein the lower beam is movable along the channel member as the drive member is moved between its retracted and advanced positions, the upper and lower beams controlling a size of the tissue gap.
16. A staple cartridge comprising:
   a body defining a central knife slot and a plurality of staple receiving slots on each side of the central knife slot;
      a plurality of pushers, each pusher of the plurality of pushers movable through one or more of the plurality of staple receiving slots; and
   a cleaner supported within the body of the staple cartridge, the cleaner positioned to clean a knife moving through the central knife slot of the body.
17. The staple cartridge of paragraph 16, wherein the cleaner includes a cleaning fluid positioned within the plurality of staple receiving slots, the cleaning fluid being forced from the plurality of staple receiving slots into the central knife slot by the plurality of pushers as the plurality of pushers move within the plurality of staple receiving slots.
18. The staple cartridge of paragraph 16, wherein the cleaner includes protrusions positioned on the body within the central knife slot.
19. The staple cartridge of paragraph 18, wherein a cleaning fluid is coated on the protrusions.
20. The staple cartridge of paragraph 19, wherein the protrusions are saturated with a cleaning fluid.

## Claims

1. A staple cartridge comprising:
a body defining a central knife slot and a plurality of staple receiving slots on each side of the central knife slot;
a plurality of pushers, each pusher of the plurality of pushers movable through one or more of the plurality of staple receiving slots; and
a cleaning fluid supported in the body of the staple cartridge, the cleaning fluid positioned to clean a knife moving through the central knife slot of the body.

2. The staple cartridge of claim 1, wherein the cleaning fluid is positioned within the plurality of staple receiving slots and is forced from the plurality of staple receiving slots into the central knife slot by the plurality of pushers as the plurality of pushers move within the plurality of staple receiving slot,

3. The staple cartridge according to claim 1 or claim 2 further including protrusions positioned on the body within the central knife slot, the protrusions supporting the cleaning fluid.

4. The staple cartridge according to any of claims 1 to 3 wherein the cleaning fluid is coated on the protrusions.

5. The staple cartridge of any of claims 1 to 3, wherein the protrusions are saturated with the cleaning fluid; preferably wherein the protrusions are in the form of brushes, wipers, or sponges.

6. A tool assembly for a surgical stapling device comprising:
an anvil;
a cartridge assembly coupled to the anvil such that the tool assembly is movable between an open position and a clamped position, the cartridge assembly including a channel member and a staple cartridge, the channel member defining a channel, the staple cartridge removably received within the channel of the channel member, the staple cartridge and the anvil defining a tissue gap in the clamped position, the staple cartridge including:
a body defining a central knife slot and a plurality of staple receiving slots on each side of the central knife slot;
a plurality of pushers, each pusher of the plurality of pushers movable through one or more of the plurality of staple receiving slots; and
a cleaning fluid supported in the body of the staple cartridge; and
a drive member supporting a knife, the drive member movable between a retracted position and an advanced position to move the knife through the central knife slot;
wherein the cleaning fluid is positioned to clean the knife as the knife moves through the central knife slot of the body.

7. The tool assembly of claim 6, wherein the cleaning fluid is positioned within the plurality of staple receiving slots and is forced from the plurality of staple receiving slots into the central knife slot by the plurality of pushers as the plurality of pushers move within the plurality of staple receiving slots; preferably further including protrusions positioned on the body within the central knife slot, the protrusions supporting the cleaning fluid.

8. The tool assembly of claim 6 or claim 7, wherein the cleaning fluid is coated on the protrusions; and/or wherein the protrusions are saturated with the cleaning fluid.

9. The tool assembly of any of claims 5 to 8, wherein the protrusions are in the form of brushes, wipers, or sponges.

10. The tool assembly of any of claims 5 to 9, wherein the drive member includes a flexible beam and a working end, the working end including an upper beam, a lower beam, and a vertical strut, the vertical strut interconnecting the upper beam and the lower beam and supporting the knife.

11. The tool assembly of any of claims 5 to 10, wherein the anvil defines a channel that receives the upper beam; preferably wherein the lower beam is movable along the channel member as the drive member is moved between its retracted and advanced positions, the upper and lower beams controlling a size of the tissue gap.

12. A staple cartridge comprising:
a body defining a central knife slot and a plurality of staple receiving slots on each side of the central knife slot;
a plurality of pushers, each pusher of the plurality of pushers movable through one or more of the plurality of staple receiving slots; and
a cleaner supported within the body of the staple cartridge, the cleaner positioned to clean a knife moving through the central knife slot of the body.

13. The staple cartridge of claim 12, wherein the cleaner includes a cleaning fluid positioned within the plurality of staple receiving slots, the cleaning fluid being forced from the plurality of staple receiving slots into the central knife slot by the plurality of pushers as the plurality of pushers move within the plurality of staple receiving slots.

14. The staple cartridge of claim 12 or claim 13, wherein the cleaner includes protrusions positioned on the body within the central knife slot.

15. The staple cartridge of claim 14, wherein a cleaning fluid is coated on the protrusions; and/or wherein the protrusions are saturated with a cleaning fluid.
